Europäisches Patentamt

European Patent Office

Office européen des brevets

(11) Publication number: **0 219 009**
**B1**

(12) **EUROPEAN PATENT SPECIFICATION**

(45) Date of publication of patent specification: **05.12.90**

(21) Application number: **86113676.0**

(22) Date of filing: **03.10.86**

(51) Int. Cl.⁵: **C 12 N 15/63,** C 12 N 1/20,
C 12 N 9/24 // (C12N15/63,
C12R1:18),(C12N15/63,
C12R1:19)

(54) Bacteria for expressing a polysaccharide depolymerase containing a novel recombinant plasmid.

(30) Priority: **07.10.85 US 784769**

(43) Date of publication of application:
**22.04.87 Bulletin 87/17**

(45) Publication of the grant of the patent:
**05.12.90 Bulletin 90/49**

(84) Designated Contracting States:
**AT BE CH DE FR GB IT LI LU NL SE**

(56) References cited:
**EP-A-0 182 106**

**CHEMICAL ABSTRACTS, vol. 104, no 23, June 9, 1986, Columbus, Ohio. USA. P A**

**VANDENBERGH et al. "Cloning and expression in Escherichia coli of the polysaccharide depolymerase associated with bacteriophage - infected Erwinia amylovora", page 228, column 2, abstract no. 201 551v**

**CHEMICAL ABSTRACTS, vol. 102, no.23, June 10, 1985, Columbus, Ohio, USA, P A**

(73) Proprietor: **MICROLIFE TECHNICS, INC.
1833 57th Street
Sarasota Florida 33578 (US)**

(72) Inventor: **Vandenbergh, Peter A.
4414 Meadowcreek Circle
Sarasota Florida 33583 (US)**

(74) Representative: **Patentanwälte Grünecker, Kinkeldey, Stockmair & Partner
Maximilianstrasse 58
D-8000 München 22 (DE)**

(56) References cited:
**VANDENBERGH et al. "Partial purification and characterization of a polysaccharide depolymerase associated with phage-infected Erwinia amylovora", page 265, column 2, abstract no. 200 072m**

**APPLIED AND ENVIRONMENTAL MICROBIOLOGY, vol. 38, no.4 October 1979 (Washington DC), A R AYERS et al. "Extracellular Polysaccharide of Erwinia amylovora : a Correlation with Virulence, pages 659-666**

**Description**

Background of the invention
(1) Field of the invention

The present invention relates to recombinant plasmids and to bacteria containing the recombinant plasmids which produce polysaccharide depolymerase, wherein the recombinant plasmid contains a DNA segment of a bacteriophage for *Erwinia amylovora*. In particular the present invention relates to *Escherichia coli* containing a recombinant plasmid with a DNA segment of phage ERA103 which expresses a depolymerase.

(2) Prior art

*Erwinia amylovora* is recognized as the causitive agent of fireblight in rosaceous plants (Ayers, A. R., et al., Appl. Environ. Microbiol. *38*:659—666 (1979)). The pathogenic strains of this bacteria have been demonstrated to produce a polysaccharide capsule which has been implicated in xylem vessel occlusion and plasmolysis of xylem parenchymal cells, which are symptomatic of the disease. U.S. Patent Application Serial No. 622,065, filed October 18, 1984 describes the use of a bacteriophage induced depolymerase from *Erwinia amylovora* for the treatment of this disease.

Bacteriophage encoded polysaccharide depolymerases have been described for many bacterial genera (Adams, M. H., et al. Virology, *2*:719—736 (1956) and Higashi, S., et al., J. Gen. Appl. Microbiol. *24*:143—153 (1978)). Bacteriophage PEal (h) has been shown to degrade the polysaccharide capsule of *Erwinia amylovora* (Hartung, J. S., et al., Phytopathology *72*:945 (1982)). Bacteriophage ERA103 has been found to infect the plant pathogen *E. amylovora* NCPPPB595 and produce a depolymerase that degrades the polysaccharide capsule of this bacteria. The problem is that it is difficult and expensive to isolate significant amounts of the depolymerase by induction with the bacteriophage.

Much more of the depolymerase might be obtained if it was possible to clone a segment of the phage DNA encoding for the depolymerase into a vector plasmid. It was uncertain whether the depolymerase could be produced without the presence of the host *Erwinia amylovora* or whether the genes responsible for encoding the depolymerase could be cloned into a recombinant plasmid.

Objects

It is therefore an object of the present invention to provide a recombinant plasmid in a bacteria which encodes for the expression of a depolymerase. Further the present invention relates to a recombinant plasmid including a segment of a bacteriophage which infects *Erwinia amylovora* and which encodes for the depolymerase. In particular it is an object of the present invention to provide a recombinant plasmid including a segment of bacteriophage ERA103 which encodes for the expression of the depolymerase in *E. coli*. Finally, it is an object of the present invention to provide a method for producing the depolymerase using the recombinant plasmid which is simple and economical. These and other objects will become increasingly apparent by reference to the following description and the drawings.

In the drawings

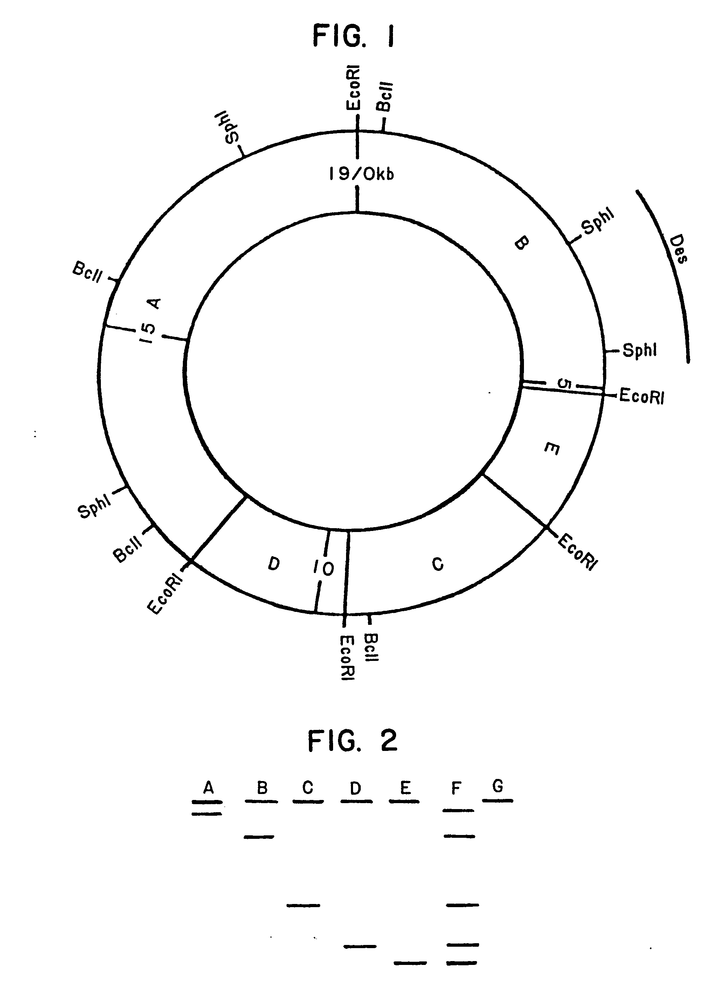

Figure 1 is a restriction map of bacteriophage ERA103 DNA wherein fragments have been lettered in order of decreasing size. *Dep* represents the fragment expressing the depolymerase activity.

Figure 2 is an agarose gel electrophoresis of DNA preparations purified with cesium chloride-ethidium bromide and digested with *Eco*RI as the restriction endonuclease enzyme. The agarose concentration was 0.7%, and migration was from top to bottom. The contents of lanes are as follows: (A) *Eco*RI digested pSRQ51; (B) *Eco*RI digested pSRQ52; (C) *Eco*RI digested pSRQ53; (D) *Eco*RI digested pSRQ54; (E) *Eco*RI digested pSRQ55; (F) *Eco*RI digested bacteriophage ERA103; (G) *Eco*RI digested pOP203($A_2^+$).

General description

The present invention relates to a recombinant plasmid which can express a polysaccharide depolymerase in bacteria containing the plasmid which comprises: a linear segment from a bacteriophage which infects *Erwinia amylovora* to produce the depolymerase and which codes for the depolymerase; and a transformable vector plasmid segment ligated to the linear segment to provide the recombinant plasmid.

Further the present invention relates to a bacteria which expresses a polysaccharide depolymerase because of a resident recombinant plasmid wherein the recombinant plasmid comprises: a linear segment from a bacteriophage which infects *Erwinia amylovora* to produce the depolymerase and which codes for the depolymerase; and a transformable vector plasmid segment ligated to the linear segment to provide the recombinant plasmid.

Finally the present invention relates to a method for producing a polysaccharide depolymerase which comprises: providing a bacteria for expressing the polysaccharide depolymerase containing a resident recombinant plasmid with a linear segment from a bacteriophage which infects *Erwinia amylovora* to produce the depolymerase and which codes for the depolymerase and with a transformable vector plasmid segment which has been ligated to the linear segment to provide the recombinant plasmid, growing the bacteria in a growth medium to produce the depolymerase; and removing the depolymerase from the bacteria and growth medium.

The preferred bacteriophage is ERA103; however other bacteriophages which induce the depolymerase in *Erwinia amylovora* which could be used are ERA225 and ERA101 which are freely available from the University of Nebraska, Dr. Anne K. Vidaver, Department of Plant Pathology.

The following specific description includes the mapping of the bacteriophage genome and the cloning of a fragment (B) from ERA103 into (1) pBR322 (Bolivar, et al., Methods of Enzymol. *68*:245—267 (1979)), (2) the positive-selection vector pOP203(A$_2$⁺) (Winter, R. B., et al., Cell *33*:877—885 (1983)), or (3) the expression vector pKK223—3 (Vievia, J., et al., Gene *19*:259—268 (1982)), as the plasmids and expression of the depolymerase in *Escherichia coli*. The positive selection vector pOP203(A$_2$⁺) contains genes that are lethal to the bacterial host. Therefore, when DNA fragments are inserted into the unique restriction sites, expression of genes deleterious to the host are suppressed. The expression vector pKK223—3 was developed for high level regulated expression of protein products in *E. coli*. This plasmid contains the strong *trp-lac (tac)* promoter which facilitates the expression of cloned genes during induction. The procedure is generally described by DeBoer, H. A., et al., P.N.A.S. *80*: 21—25 (1983).

Specific description
Materials and methods
*Bacterial strains and media*

Bacterial strains used or constructed in this invention are presented in Table 1.

TABLE 1
Bacterial strains and plasmids

| Strain bacteria | Remarks | Reference |
|---|---|---|
| *E. coli* | | |
| HB101 | *lacl*[qa] | 1 |
| HB101 (pOP203A$_{2+}$) | contains positive selection vector | 1 |
| HB101 (pBR322) | contains plasmid pBR322 | 4 |
| JM105 | *lacl*[q] | 3 |
| JM105 (pKK223—3) | contains expression vector | 2 |
| Plasmids | | |
| pOP203(A$_2$+) *EcoR*I site | *lac*[b] *A$_2$*[c] *tet*[d] | 1 |
| pBR322 | *bla*[3] *tet* | 4 |
| pKK223—3 | *bla tet tac*[f] | 3 |
| pSRQ51 | phage fragment A in pOP203(A$_2$+)*EcoR*I site | present invention |
| pSRQ52 | phage fragment B in pOP203(A$_2$+) *EcoR*I site | NRRL-B-15989 |
| pSRQ53 | phage fragment C in pOP203(A$_2$+) *EcoR*I site | present invention |
| pSRQ54 | phage fragment D in pOP203(A$_2$+) *EcoR*I site | present invention |
| pSRQ55 | phage fragment E in pOP203(A$_2$+) *EcoR*I site | present invention |
| pSRQ56 | phage fragment B in pBR322 *EcoR*I site | NRRL-B-15990 |
| pSRQ57 | phage fragment B in pKK223—3 *EcoR*I site | NRRL-B-15991 |
| pSRQ58 | *Sph*I segment of the B fragment in pBR322 *Sph*I site | present invention |

[a]hyperlactose repressor-producing mutation carried by F'*lac* exogenate.
[b]lactose promoter/operator.
[c]maturation protein gene of the RNA bacteriophage Qbeta.
[d]tetracycline resistance.
[e]ampicillin resistance.
[f]*trp-lac* promoter.

[1]Winter, R. B., et al. Cell *33*:877—885 (1983).
[2] DeBoer, H. A., et la. P.N.A.S. *80*:21—25 (1983).
[3]Vievia, J., et al. Gene *19*:259—268 (1982).
[4]Bolivar, F., et al., Methods Enzymol. *68*:245—267 (1979).

*E. coli* strains were grown in L broth (Davis, R. W., et al., A manual for genetic engineering: advanced bacterial genetics, Cold Spring Harbor Laboratory, Cold Spring Harbor, N.Y. (1980)). Tetracycline (Sigma Chemical Co., St. Louis, MO.) was added to media at a concentration of 10 micrograms/ml. Carbenicillin (United States Biochemical Corporation, Cleveland, OH.) was added to media at a concentration of 50 micrograms/ml. Isopropyl B-D-thiogalactoside (IPTG; Sigma) was added to the media for a final concentration of 1.0 mM where indicated.

## Bacteriophage preparation

High titer stocks of bacteriophage ERA103 ($10^{11}$ to $10^{12}$ PFU/ml) were developed as previously described (Yamamoto, K. R., et al., Virology *40*:734—744 (1970)).

## Plasmid and bacteriophage DNA isolation

Plasmid DNA was isolated from *E. coli* by a method previously described in the literature (Vandenbergh, P. A., et al., J. Dent. Res. *61*:497—501 (1982)). Polyethylene glycol (PEG) precipitated high titer phage preparations were centrifuged in cesium chloride-ethidium bromide gradients. The cesium chloride purified bacteriophage DNA was dialyzed overnight in 4.0 l of 0.01 M tris (hydroxymethyl) aminomethane-hydrochloride (pH 8.0) containing 0.001 M ethylenediaminetetracetic acid. Subsequently, the bacteriophage DNA preparation was extracted twice with saturated phenol and ethanol precipitated.

## Bacterial transformations

*E. coli* was transformed by the $CaCl_2$-heat shock method (Davis, R. W., et al. A manual for genetic engineering: advanced bacterial genetics, Cold Spring Harbor Laboratory, Cold Spring Harbor, N.Y. (1980)), with cells harvested at an absorbance of 0.6 (600 nm).

## Restriction endonuclease digestion and ligation

Restriction endonucleases and T4 DNA ligase (Bethesda Research Laboratories, Bethesda, MD.) were used in the buffers and under the conditions recommended by the supplier.

## Construction and screening of clones

Bacteriophage ERA103 DNA was digested with *Eco*RI and ligated with pOP203($A_2^+$) or pBR322 vectors cut to completion with *Eco*RI. The ligation mixture contained approximately a 1:2 ratio of vector DNA to bacteriophage DNA. Bacteriophage ERA103 DNA was digested with *Eco*RI and ligated with pKK223—3 vector cut to completion with *Eco*RI. The ligation mixture contained approximately a 1:10 ratio of vector DNA to bacteriophage DNA. Ligation was performed at 17°C for 18 hours. The ligation procedure uses DNA T4 ligase as described by *Bolivar* et al. (1979).

## Determination of depolymerase activity

Bacterial cultures were grown overnight in L broth at 37°C supplemented with either tetracycline at a concentration of 10 micrograms/ml, or carbenicillin at a concetration of 50 micrograms/ml depending on the vector utilized. This culture was then diluted 1:100 into 250 ml of similar media with cells harvested at an absorbance of 0.9 (600 nm). All subsequent fractionation steps were performed at 4°C. The cells were then centrifuged at 8,000 xg for 30 min. The pellet was then suspended in sterile distilled water and centrifuged at 8,000 xg for 30 minutes. The washed pellet was then resuspended in 10 ml of 0.01 M citrate buffer (pH 6.0) containing 0.01M 2-mercaptoethanol. Cell-free extracts (CFE) were prepared by passage of washed cell suspensions through a French Press at 16,000 p.s.i., three times. The resultant CFE was then centrifuged at 27,000 xg for 30 minutes to remove whole cells and cell debris. Ammonium sulfate was added slowly to the supernatant to give a final concentration of 45% saturation and precipitated for 18 hours. The ammonium sulfate precipitate was centrifuged at 27,000 xg for 30 minutes and dialyzed overnight against buffer. Depolymerase activity was assayed by following the release of galactose from the polysaccharide substrate by the method of Fairbridge et al. (Fairbridge, R. A., et al., Biochem. J. *49*:423—427 (1951)). Polysaccharide was prepared from uninfected cultures of *E. amylovora* NCPPB595 cultivated on sheets of cellophane overlaying tryptic soy agar, as described by Liu et al. (Liu, P. V., et al., J. Infect. Dis. *108*:218—228 (1961)).

One unit of enzyme is the amount of enzyme to produce 1 micromole of galactose equivalent per minute under standard assay conditions. Protein concentrations were determined by the method of Koch and Putnam (Koch, A., et al., Anal. Biochem. *44*:239—245 (1971)).

## Induction studies

Bacterial culture were grown overnight in L broth at 37°C supplemented with the appropriate antibiotic. This culture was then diluted 1:100 into 250 ml of similar media until the cells reached an absorbance of 0.7 (600 nm). IPTG was added to a final concentration of 1 mM, and incubated until an absorbance of 0.9 (600 nm) was obtained. The cultures were then processed as described in the previous section.

## Preparation of depolymerase

Figure 1 which is a restriction map depicts the relative positions of various restriction endonuclease recognition sites on phage ERA103 DNA obtained using a combination of the following procedures: (i) analysis of DNA fragments obtained after digestion with two enzymes; (ii) analysis of *E. coli* plasmids containing phage ERA103 *Eco*RI fragments. DNA from phage ERA103 was cleaved by *Eco*RI into five distinct fragments labelled: A, 7.5-kb; B, 5.0-kb; C, 2.7-kb; D, 2.1-kb and E, 1.8-kb. Several restriction enzymes: *Bam*HI, *Bst*EII, *Cla*I, *Hind*III, *Kpn*I, *Pst*I, *Sal*I and *Sst*I, did not cleave the phage DNA.

The plasmid pOP203($A_2^+$) contains the *lac* promoter-operator fused to the Q beta phage maturation gene. Positive selection results when only survivors with inserts in the $A_2$ gene grow in the presence of IPTG. Since the vector has several unique restriction sites, including *EcoR*I, this vector was used. Colonies were then screened for the presence of plasmid DNA containing the five *EcoR*I phage fragments. All five *EcoR*I fragments were cloned into pOP203(A$_2$$^+$) (Fig. 2). The plasmid containing strains were grown in 250 ml of L broth containing tetracycline at 10 micrograms/ml. Enzyme assays in triplicate of CFE of each cloned fragment demonstrated that enzyme activity was associated with the B fragment which is 5.0-kb, and contained in pSRQ52. Depolymerase activity was always associated with the supernatants of the CFE and not demonstrated in the pellets.

Mapping data revealed the presence of *Sph*I sites in the phage ERA103 DNA. The vector pBR322 was utilized because of the insertional inactivation of tetracycline in the *Sph*I site of this vector. Transformants that were resistant to carbenicillin and sensitive to tetracycline were screened for plasmids and depolymerase activity. An *E. coli* strain containing the plasmid pSRQ58, a 1.5-kb portion of the B fragment demonstrated depolymerase activity.

Additional cloning experiments utilized the expression vector pKK223—3. The B fragment was cloned in the *EcoR*I site of this plasmid.

Expression of the enzyme was examined utilizing various *E. coli* strains containing the cloned depolymerase fragment as shown in Table 2.

TABLE 2
Depolymerase activity by *E. coli* HB101 containing various plasmids

| Plasmid | Growth conditions | Enzyme sp. act.[a] |
|---|---|---|
| None | Uninduced | 0[b] |
| pBR322 | Uninduced | 0 |
| pSRQ56 | Uninduced | 27.1 |
| pSRQ58 | Uninduced | 39.0 |
| pOP203(A$_2$$^+$) | Uninduced | 0 |
| pOP203(A$_2$$^+$) | Induced | 0 |
| pSRQ52 | Uninduced | 28.0 |
| pSRQ52 | Induced | 34.0 |
| pKK223—3 | Uninduced | 0 |
| pKK223—3 | Induced | 0 |
| pSRQ57 | Uninduced | 34.0 |
| pSRQ57 | Induced | 47.2 |

[a] Specific activities are expressed in micromole of galactose equivalent per minute per milligram of protein under standard assay conditions.
[b] A measurement of zero implies activity of <0.05.

The *E. coli* strains containing pSRQ57 or pSRQ52 were grown and induced with IPTG or uninduced. The *E. coli* strains containing pSRQ56 or pSRQ58 were also grown and compared to the above *E. coli* strains. The CFE supernatants were precipitated with ammonium sulfate and dialyzed against buffer. All of the ammonium sulfate precipitates were standardized to equivalent protein concentrations and assayed for depolymerase in triplicate. The *E. coli* strains containing plasmids with the *tac* promoter demonstrated twenty additional depolymerase units upon induction. Strains containing plasmids with only the *lac* promoter demonstrated seven additional units when induced. Table 2 demonstrates that the gene coding for the depolymerase from the bacteriophage ERA103 and related bacteriophages can be cloned into pBR322, pOP203(A$_2$$^+$), pKK223—3 and expressed in *E. coli*. It can also be expressed with other vectors and bacteria as will be obvious to those skilled in the art.

The physical map of ERA103 in Figure 1 includes the positions for cleavage sites of three restriction enzymes. Enzyme assays conducted on the cell free extract (CFE) supernatants of the five cloned *EcoR*I fragments of bacteriophage DNA demonstrated that the depolymerase activity was associated with the 5.0-kb B fragment. Subsequent subcloning utilizing pBR322, associated the activity within a 1.5-kb *Sph*I fragment. When the depolymerase activity of entire cloned B fragment in either pBR322 or uninduced pOP203(A₂⁺) was compared to the activity of the subcloned *Sph*I fragment, the activity was 69% greater in the subcloned *Sph*I fragment. Induction utilizing the entire B fragment cloned in the expression vector pKK223—3 demonstrated a 77% increase in enzymatic activity compared to the same fragment cloned into pBR322. Use of the positive selection vector pOP203(A₂⁺) resulted in a 27% increase in activity compared to pBR322.

The plasmid pOP203(A₂⁺) was derived from pOP203—3, a pMB9 plasmid carrying the *lac* UV5 promoter (Fuller, F., Gene *19*:43—54 (1982)). The expression vector pKK223—3 contains the *lac*UV5 promoter and the *trp* promoter, i.e. *tac* (Amann, E., et al., Gene *25*:167—178 (1983)). The plasmid pBR322 does not have a *lac* promoter. Enzymatic assays indicated that the expression level varied with the promoter utilized.

The polysaccharide depolymerase is a replacement for antibiotic therapy in the control of *E. amylovora*, the causative agent of Fireblight. Plants were treated with the depolymerase alone or as shown in U.S. patent application Serial No. 622,056, filed October 18, 1984. The depolymerase can be combined with various leaf wetting agents or polymeric adhesives to cause the enzyme to adhere to the plant.

The foregoing specific description is only illustrative of the present invention and it is intended that the present invention be limited only by the hereinafter appended claims.

**Claims for the Contracting States: BE CH DE FR GB IT LI LU NL SE**

1. A recombinant plasmid which can express a polysaccharide depolymerase in bacteria containing the plasmid which comprises:
(a) a linear segment from a bacteriophage which infects *Erwinia amylovora* to produce the depolymerase and which codes for the depolymerase; and
(b) a transformable vector plasmid segment ligated to the linear segment to provide the recombinant plasmid.

2. The plasmid of Claim 1 wherein the linear segment is from bacteriophage ERA103 deposited as ATCC 39824 B1.

3. The recombinant plasmid of Claim 1 as carried in *E. coli* NRRL-B-15989.

4. The recombinant plasmid of Claim 1 as carried in *E. coli* NRRL-B-15990.

5. The recombinant plasmid of Claim 1 as carried in *E. coli* NRRL-B-15991.

6. The recombinant plasmid of Claim 1 wherein the linear segment is from ERA103 deposited as ATCC 39824 B1 cleaved by endonuclease *Eco*Ri, *Sph*I, or *Eco*RI and then *Sph*I and the vector plasmid is selected from pOP203(A₂⁺), pBR322 and pKK223—3 cleaved by *Eco*RI, which has been ligated to the linear segment to provide the recombinant plasmid.

7. The recombinant plasmid of Claim 1 wherein the bacteria is *E. coli*.

8. The plasmid of Claim 1 wherein the vector plasmid and linear segment have been cleaved by *Eco*RI and ligated.

9. The plasmid of Claim 1 wherein the phage lyses *Erwinia amylovora* and wherein the polysaccharide depolymerase degrades a polysaccharide capsule around the *Erwinia amylovora*.

10. The plasmid of Claim 9 wherein the *Erwinia amylovora* is deposited as ATCC 39824.

11. A bacterium which expresses a polysaccharide depolymerase because of a resident recombinant plasmid wherein the recombinant plasmid comprises:
(a) a linear segment from a bacteriophage which infects *Erwinia amylovora* to produce the depolymerase and which codes for the depolymerase; and
(b) a transformable vector plasmid segment ligated to the linear segment to provide the recombinant plasmid.

12. The bacterium of Claim 11 wherein the linear segment is from bacteriophage ERA103 as carried in ATCC39824 B1.

13. The bacterium of Claim 11 containing the recombinant plasmid as carried in *Escherichia coli* NRRL-B-15990.

14. The bacterium of Claim 11 containing the recombinant plasmid carried in *Escherichia coli* NRRL-B-15991.

15. The bacterium of Claim 11 containing the recombinant plasmid as carried in *E. coli* NRRL-B-15989 (for pSRQ52).

16. The bacterium of Claim 11 wherein the linear segment is from ERA103 cleaved by endonuclease *Eco*RI, *Sph*I or *Eco*RI and then *Sph*I and the vector plasmid is selected from pOP203 (A₂⁺), pBR322 and pKK223—3 cleaved by *Eco*RI and which has been ligated to the linear segment to provide the recombinant plasmid.

17. The bacterium of Claim 11 which is a strain of *Escherichia coli*.

18. The bacterium of Claim 11 wherein the vector plasmid and the linear segment have been cleaved by *Eco*RI and ligated.

19. The bacterium of Claim 11 wherein the phage lyses *Erwinia amylovora* and wherein the polysaccharide depolymerase degrades a polysaccharide capsule around the *Erwinia amylovora*.

20. The bacterium of Claim 19 wherein the *Erwinia amylovora* is ATCC 39824.

21. A method for producing a polysaccharide depolymerase which comprises:

(a) providing a bacterium for expressing the polysaccharide polymerase containing a resident recombinant plasmid with a linear segment from a bacteriophage which infects *Erwinia amylovora* to produce the depolymerase and which codes for the depolymerase and with a transformable vector plasmid segment which has been ligated to the linear segment to provide the recombinant plasmid;

(b) growing the bacterium in a growth medium to produce the depolymerase; and

(c) removing the depolymerase from the bacteria and growth medium.

22. The method of Claim 21 wherein cells of the bacterium are ruptured and the depolymerase is removed from the ruptured cells.

23. The method of Claim 21 wherein the linear segment is from bacteriophage ERA103 deposited as ATCC 39824 B1.

24. The method of Claim 21 wherein the bacterium is *Escherichia coli*.

25. The method of Claim 21 wherein the recombinant plasmid is carried in *Escherichia coli* NRRL-B-15989.

26. The method of Claim 21 wherein the recombinant plasmid is carried in *Escherichia coli* NRRL-B-15990.

27. The method of Claim 21 wherein the recombinant plasmid is carried in *E. coli* NRRL-B-15991.

28. The method of Claim 21 wherein the linear segment is from ERA103 ATCC 39824 B1 cleaved by endonuclease *Eco*RI, *Sph*I or *Eco*RI and then *Sph*I and the vector plasmid is selected from pOP203 ($A_2^+$), pBR322 and pKK223—3 cleaved by *Eco*RI and then ligated to provide the recombinant plasmid.

29. The method of Claim 21 wherein the phage lyses *Erwinia amylovora* and wherein the depolymerase degrades a polysaccharide capsule of the *Erwinia amylovora*.

30. The method of Claim 29 wherein the *Erwinia amylovora* is ATCC 39824.

**Claims for the Contracting State: AT**

1. A process for the preparation of a recombinant plasmid which can express a polysaccharide depolymerase in bacteria containing the plasmid which comprises combining:

(a) a linear segment from a bacteriophage which infects *Erwinia amylovora* to produce the depolymerase and which codes for the depolymerase; and

(b) a transformable vector plasmid segment ligated to the linear segment to provide the recombinant plasmid.

2. The process of Claim 1 wherein the linear segment is from bacteriophage ERA103 deposited as ATCC 39824 B1.

3. The process of claim 1, resulting in a recombinant plasmid as carried in *E. coli* NRRL-B-15989.

4. The process of Claim 1, resulting in a recombinant plasmid as carried in *E. coli* NRRL-B-15990.

5. The process of Claim 1, resulting in a recombinant plasmid as carried in *E. coli* NRRL-B-15991.

6. The process of Claim 1 wherein the linear sgement is from ERA103 deposited as ATCC 39824 B1 cleaved by endonuclease *Eco*RI, *Sph*I, or *Eco*RI and then *Sph*I and the vector plasmid is selected from pOP203($A_2^+$), pBR322 and pKK223—3 cleaved by *Eco*RI, which has been ligated to the linear segment to provide the recombinant plasmid.

7. The process of Claim 1 wherein the bacterium is *E. coli*.

8. The process of Claim 1 wherein the vector plasmid and linear segment are cleaved by *Eco*RI and ligated.

9. The process of Claim 1 wherein the phage lyses *Erwinia amylovora* and wherein the polysaccharide depolymerase degrades a polysaccharide capsule around the *Erwinia amylovora*.

10. The process of Claim 9 wherein the *Erwinia amylovora* is deposited as ATCC 39824.

11. A process for the preparation of a bacterium which expresses a polysaccharide depolymerase, wherein a recombinant plasmid is introduced in said bacterium, wherein the recombinant plasmid comprises:

(a) a linear segment from a bacteriophage which infects *Erwinia amylovora* to produce the depolymerase and which codes for the depolymerase; and

(b) a transformable vector plasmid segment ligated to the linear segment to provide the recombinant plasmid.

12. The process of Claim 11 wherein the linear segment is from bacteriophage ERA103 as carried in ATCC 39824 B1.

13. The process of Claim 11 wherein the recombinant plasmid is the plasmid as carried in *Escherichia coli* NRRL-B-15990.

14. The process of Claim 11 wherein the recombinant plasmid is the plasmid as carried in *Escherichia coli* NRRL-B-15991.

15. The process of Claim 11 wherein the recombinant plasmid is the plasmid as carried in *E. coli* NRRL-B-15989.

16. The process of Claim 11 wherein the linear segment is from ERA103 cleaved by endonuclease *Eco*RI, *Sph*I or *Eco*RI and then *Sph*I and the vector plasmid is selected from pOP203($A_2^+$), pBR322 and

pKK223—3 cleaved by *Eco*RI and which has been ligated to the linear segment to provide the recombinant plasmid.

17. The process of Claim 11 wherein the bacterium is a strain of *Escherichia coli.*

18. The process of Claim 11 wherein the vector plasmid and the linear segment have been cleaved by *Eco*RI and ligated.

19. The process of Claim 11 wherein the phage lyses *Erwinia amylovora* and wherein the polysaccharide depolymerase degrades a polysaccharide capsule around the *Erwinia amylovora.*

20. The process of Claim 19 wherein the *Erwinia amylovora* is ATCC 39824.

21. A method for producing a polysaccharide depolymerase which comprises:

(a) providing bacteria for expressing the polysaccharide polymerase containing a resident recombinant plasmid with a linear segment from a bacteriophage which infects *Erwinia amylovora* to produce the depolymerase and which codes for the depolymerase and with a transformable vector plasmid segment which has been ligated to the linear sgement to provide the recombinant plasmid;

(b) growing the bacteria in a growth medium to produce the depolymerase; and

(c) removing the depolymerase from the bacteria and growth medium.

22. The method of Claim 21 wherein cells of the bacteria are ruptured and the depolymerase is removed from the ruptured cells.

23. The method of Claim 21 wherein the linear segment is from bacteriophage ERA103 deposited as ATCC 39284 B1.

24. The method of Claim 21 wherein the bacterium is *Escherichia coli.*

25. The method of Claim 21 wherein the recombinant plasmid is carried in *Escherichia coli* NRRL-B-15989.

26. The method of Claim 21 wherein the recombinant plasmid is carried in *Escherichia coli* NRRL-B-15990.

27. The method of Claim 21 wherein the recombinant plasmid is carried in *E. coli* NRRL-B-15991.

28. The method of Claim 21 wherein the linear segment is from ERA103 ATCC 39824 B1 cleaved by endonuclease *Eco*RI, *Sph*I or *Eco*RI and then *Sph*I and the vector plasmid is selected from pOP203(A$_2$+), pBR322 and pKK223—3 cleaved by *Eco*RI and then ligated to provide the recombinant plasmid.

29. The method of Claim 21 wherein the phage lyses *Erwinia amylovora* and wherein the depolymerase degrades a polysaccharide capsule of the *Erwinia amylovora.*

30. The method of Claim 22 wherein the *Erwinia amylovora* is ATCC 39824.

**Patentansprüche für die Vertragsstaaten: BE CH DE FR GB IT LI LU NL SE**

1. Rekombinantes Plasmid, das eine Polysaccharid-Depolymerase in Bakterien, die das Plasmid enthalten, exprimieren kann, umfassend

(a) ein lineares Fragment eines Bakteriophagen, der *Erwinia amylovora* infiziert, um die Depolymerase zu erzeugen, und das für die Depolymerase kodiert; und

(b) ein Fragment eines transformierbaren Vektorplasmides, das mit dem linearen Fragment ligiert ist, um das rekombinante Plasmid bereitzustellen.

2. Plasmid nach Anspruch 1, dadurch gekennzeichnet, daß das lineare Fragment aus dem mit der Hinterlegungsnummer ATCC 39824 B1 hinterlegtem Bakteriophagen ERA103 stammt.

3. Rekombinantes Plasmid nach Anspruch 1, wie es in *E. coli* NRRL-B-15989 enthalten ist.

4. Rekombinantes Plasmid nach Anspruch 1, wie es in *E. coli* NRRL-B-15990 enthalten ist.

5. Rekombinantes Plasmid nach Anspruch 1, wie es in *E. coli* NRRL-B-15991 enthalten ist.

6. Rekombinantes Plasmid nach Anspruch 1, dadurch gekennzeichnet, daß das lineare Fragment aus dem als ATCC 39824 B1 hinterlegtem ERA103 stammt, der mit der Endonuklease *Eco*RI, *Sph*I, oder *Eco*RI und anschließend *Sph*I geschnitten worden ist, und daß das Vektorplasmid aus der pOP203(A$_2$+), pBR322 und pKK223—3 umfassenden Gruppe ausgewählt ist, wobei das Vektorplasmid mit *Eco*RI gespalten und mit dem linearen Fragment ligiert worden ist, um das rekombinante Plasmid bereitzustellen.

7. Rekombinantes Plasmid nach Anspruch 1, dadurch gekennzeichnet, daß das Bakterium *E. coli* ist.

8. Plasmid nach Anspruch 1, dadurch gekennzeichnet, daß das Vektorplasmid und das lineare Fragment mit *Eco*RI (heraus)gespalten und ligiert worden sind.

9. Plasmid nach Anspruch 1, dadurch gekennzeichnet, daß der Phage *Erwinia amylovora* lysiert und die Polysaccharid-Depolymerase eine Polysaccharidkapsel um *Erwinia amylovora* abbaut.

10. Plasmid nach Anspruch 9, dadurch gekennzeichnet, daß *Erwinia amylovora* als ATCC 39824 hinterlegt worden ist.

11. Bakterium, das eine Polysaccharid-Depolymerase aufgrund eines darin seßhaften rekombinanten Plasmides exprimiert, wobei das rekombinante Plasmid umfaßt:

(a) ein lineares Fragment eines Bakteriophagen, der *Erwinia amylovora* infiziert, um die Depolymerase zu erzeugen, und für die Depolymerase kodiert; und

(b) ein Fragment eines transformierbaren Vektorplasmides, das mit dem linearen Fragment ligiert ist, um das rekombinante Plasmid bereitzustellen.

12. Bakterium nach Anspruch 11, dadurch gekennzeichnet, daß das lineare Fragment aus dem Bakteriophagen ERA103, wie er in ATCC 39824 B1 enthalten ist, stammt.

13. Bakterium nach Anspruch 11, dadurch gekennzeichnet, daß es das rekombinante Plasmid enthält, wie es in *Escherichia coli* NRRL-B-15990 enthalten ist.

14. Bakterium nach Anspruch 11, dadurch gekennzeichnet, daß es das rekombinante Plasmid enthält, das in *Escherichia coli*, NRRL-B-15991 enthalten ist.

15. Bakterium nach Anspruch 11, dadurch gekennzeichnet, daß es das rekombinante Plasmid enthält, wie es in *E. coli* NRRL-B-15989 (für pSRQ52) enthalten ist.

16. Bakterium nach Anspruch 11, dadurch gekennzeichnet, daß das lineare Fragment aus ERA103 stammt, der mit der Endonuklease *Eco*RI, *Sph*I oder *Eco*RI und anschließend *Sph*I gespalten worden ist, und das Vektorplasmid aus der pOP203(A$_2$$^+$), pBR322 und pKK223—3 umfassenden Gruppe ausgewählt ist, wobei das Vektorplasmid mit *Eco*RI gespalten und mit dem linearen Fragment ligiert worden ist, um das rekombinante Plasmid bereitzustellen.

17. Bakterium nach Anspruch 11, dadurch gekennzeichnet, daß es ein *Escherichia coli*-Stamm ist.

18. Bakterium nach Anspruch 11, dadurch gekennzeichnet, daß das Vektorplasmid und das lineare Fragment mit *Eco*RI (heraus)gespalten und ligiert worden sind.

19. Bakterium nach Anspruch 11, dadurch gekennzeichnet, daß der Phage *Erwinia amylovora* lysiert und die Polysaccharid-Depolymerase eine Polysaccharidkapsel rund um *Erwinia amylovora* abbaut.

20. Bakterium nach Anspruch 19, dadurch gekennzeichnet, daß es *Erwinia amylovora* ATCC 39824 ist.

21. Verfahren zum Herstellen einer Polysaccharid-Depolymerase, umfassend:

(a) Bereitstellen eines Bakteriums zum Exprimieren der Polysaccharid-Polymerase, das ein darin seßhaftes rekombinantes Plasmid mit einem linearen Fragment eines Bakteriophagen, der *Erwinia amylovora* zum Herstellen der Depolymerase, infiziert, enthält, das für die Depolymerase kodiert, mit einem Fragment eines transformierbaren Vektorplasmides, das mit dem linearen Fragment ligiert worden ist, um ein rekombinantes Plasmid bereitzustellen;

(b) Wachsenlassen des Bakteriums in einem Wachstumsmedium, um die Depolymerase herzustellen; und

(c) Entfernen der Depolymerase aus dem Bakterium und Wachstumsmedium.

22. Verfahren nach Anspruch 21, dadurch gekennzeichnet, daß die Zellen des Bakteriums aufgebrochen und die Depolymerase aus den aufgebrochenen Zellen entfernt wird.

23. Verfahren nach Anspruch 21, dadurch gekennzeichnet, daß das lineare Fragment aus dem als ATCC 39824 B1 hinterlegten Bakteriophagen ERA103 stammt.

24. Verfahren nach Anspruch 21, dadurch gekennzeichnet, daß das Bakterium *Escherichia coli* ist.

25. Verfahren nach Anspruch 21, dadurch gekennzeichnet, daß das rekombinante Plasmid in *Escherichia coli* NRRL-B-15989 enthalten ist.

26. Verfahren nach Anspruch 21, dadurch gekennzeichnet, daß das rekombinante Plasmid in *Escherichia coli* NRRL-B-15990 enthalten ist.

27. Verfahren nach Anspruch 21, dadurch gekennzeichnet, daß das rekombinante Plasmid in *E. coli* NRRL-B-15991 enthalten ist.

28. Verfahren nach Anspruch 21, dadurch gekennzeichnet, daß das lineare Fragment aus ERA103 ATCC 39824 B1 stammt, der mit der Endonuklease *Eco*RI, *Sph*I oder *Eco*RI und anschließend *Sph*I gespalten worden ist, und das Vektorplasmid aus der pOP203(A$_2$$^+$), pBR322 und pKK223—3 umfassenden Gruppen ausgewählt ist, wobei das Vektorplasmid mit *Eco*RI gespalten und dann ligiert worden ist, um das rekombinante Plasmid bereitzustellen.

29. Verfahren nach Anspruch 21, dadurch gekennzeichnet, daß der Phage *Erwinia amylovora* lysiert und die Depolymerase eine Polysaccharidkapsel von *Erwinia amylovora* abbaut.

30. Verfahren nach Anspruch 29, dadurch gekennzeichnet, daß der *Erwinia amylovora* ATCC 39824 ist.

**Patentansprüche für den Vertragsstaat: AT**

1. Verfahren zum Herstellen eines rekombinanten Plasmides, das eine Polysaccharid-Depolymerase in Bakterien, die das Plasmid enthalten, exprimieren kann, umfassend das Verbinden von:

(a) einem linearen Fragment von einem Bakteriophagen, der *Erwinia amylovora* infiziert, um eine Depolymerase zu erzeugen, und der für die Depolymerase kodiert; und

(b) einem Fragment eines transformierbaren Vektorplasmides, das mit dem linearen Fragment ligiert wird, um ein rekombinantes Plasmid bereitzustellen.

2. Verfahren nach Anspruch 1, dadurch gekennzeichnet, daß das lineare Fragment aus dem als ATCC 39824 B1 hinterlegtem Bakteriophagen ERA103 stammt.

3. Verfahren nach Anspruch 1, das zu einem rekombinanten Plasmid, wie es in *E. coli* NRRL-B-15989 enthalten ist, führt.

4. Verfahren nach Anspruch 1, dadurch gekennzeichnet, daß es zu einem rekombinanten Plasmid, wie es in *E. coli* NRRL-B-15990 enthalten ist, führt.

5. Verfahren nach Anspruch 1, dadurch gekennzeichnet, daß es zu einem rekombinanten Plasmid, wie es in *E. coli* NRRL-B-15991 enthalten ist, führt.

6. Verfahren nach Anspruch 1, dadurch gekennzeichnet, daß das lineare Fragment aus dem als ATCC 39824 B1 hinterlegtem ERA103 stammt, der mit der Endonuklease *Eco*RI, *Sph*I, oder *Eco*RI und anschließend *Sph*I gespalten worden ist, und das Vektorplasmid aus der pOP203(A$_2$$^+$), pBR322 und

pKK223—3 umfassenden Gruppe ausgewählt ist, wobei das Vektorplasmid mit *Eco*RI gespalten und mit dem linearen Fragment ligiert worden ist, um ein rekombinantes Plasmid bereitzustellen.

7. Verfahren nach Anspruch 1, dadurch gekennzeichnet, daß das Bakterium *E. coli* ist.

8. Verfahren nach Anspruch 1, dadurch gekennzeichnet, daß das Vektorplasmid und das lineare Fragment mit *Eco*RI gespalten und ligiert werden.

9. Verfahren nach Anspruch 1, dadurch gekennzeichnet, daß der Phage *Erwinia amylovora* lysiert und die Polysaccharid-Depolymerase eine Polysaccharidkapsel rund um den *Erwinia amylovora* abbaut.

10. Verfahren nach Anspruch 9, dadurch gekennzeichnet, daß der *Erwinia amylovora* als ATCC 39824 hinterlegt ist.

11. Verfahren zum Herstellen eines Bakteriums, das eine Polysaccharid-Depolymerase exprimiert, dadurch gekennzeichnet, daß ein rekombinantes Plasmid in das Bakterium eingeführt wird, wobei das rekombinante Plasmid umfaßt:

(a) ein lineares Fragment aus einem Bakteriophagen, der *Erwinia amylovora* infiziert, um eine Depolymerase zu erzeugen, und für die Depolymerase kodiert; und

(b) ein Fragment eines transformierbaren Vektorplasmides, das mit dem linearen Fragment ligiert ist, um ein rekombinantes Plasmid bereitzustellen.

12. Verfahren nach Anspruch 11, dadurch gekennzeichnet, daß das lineare Fragment aus dem Bakteriophagen ERA103 stammt, wie er in ATCC 39824 B1 enthalten ist.

13. Verfahren nach Anspruch 11, dadurch gekennzeichnet, daß das rekombinante Plasmid das Plasmid ist, wie es in *Escherichia coli* NRRL-B-15990 enthalten ist.

14. Verfahren nach Anspruch 11, dadurch gekennzeichnet, daß das rekombinante Plasmid das Plasmid ist, wie es in *Escherichia coli* NRRL-B-15991 enthalten ist.

15. Verfahren nach Anspruch 11, dadurch gekennzeichnet, daß das rekombinante Plasmid das Plasmid ist, wie es in *E. coli* NRRL-B-15989 enthalten ist.

16. Verfahren nach Anspruch 11, dadurch gekennzeichnet, daß das lineare Fragment aus ERA103 stammt, der mit der Endonuklease *Eco*RI, *Sph*I oder *Eco*RI und anschließend *Sph*I gespalten worden ist, und das Vektorplasmid aus der pOP203($A_2^+$), pBR322 und pKK223—3 umfassenden Gruppe ausgewählt ist, wobei das Vektorplasmid mit *Eco*RI gespalten und dann mit dem linearen Fragment ligiert worden ist, um ein rekombinantes Plasmid bereitzustellen.

17. Verfahren nach Anspruch 11, dadurch gekennzeichnet, daß das Bakterium ein *Escherichia coli*-Stamm ist.

18. Verfahren nach Anspruch 11, dadurch gekennzeichnet, daß das Vektorplasmid und das lineare Fragment mit *Eco*RI gespalten und ligiert worden sind.

19. Verfahren nach Anspruch 11, dadurch gekennzeichnet, daß der Phage *Erwinia amylovora* lysiert und die Polysaccharid-Depolymerase eine Polysaccharidkapsel rund um *Erwinia amylovora* abbaut.

20. Verfahren nach Anspruch 19, dadurch gekennzeichnet, daß der *Erwinia amylovora* ATCC 39824 ist.

21. Verfahren zum Herstellen einer Polysaccharid-Depolymerase, umfassend:

(a) Bereitstellen von Bakterien zum Exprimieren der Polysaccharid-Polymerase, die ein darin seßhaftes rekombinantes Plasmid mit einem linearen Fragment aus einem Bakteriophagen, der *Erwinia amylovora* zum Herstellen der Depolymerase infiziert, enthalten, das für die Depolymerase kodiert, mit einem Fragment eines transformierbaren Vektorplasmides, das mit dem linearen Fragment ligiert worden ist, um das rekombinante Plasmid bereitzustellen;

(b) Wachsenlassen des Bakteriums in einem Wachstumsmedium, um die Depolymerase herzustellen; und

(c) Entfernen der Depolymerase aus dem Bakterium und dem Wachstumsmedium.

22. Verfahren nach Anspruch 21, dadurch gekennzeichnet, daß Zellen der Bakterien aufgebrochen werden und die Depolymerase aus den aufgebrochenen Zellen entfernt wird.

23. Verfahren nach Anspruch 21, dadurch gekennzeichnet, daß das lineare Fragment aus dem als ATCC 39824 B1 hinterlegtem Bakteriophagen ERA103 stammt.

24. Verfahren nach Anspruch 21, dadurch gekennzeichnet, daß das Bakterium *Escherichia coli* ist.

25. Verfahren nach Anspruch 21, dadurch gekennzeichnet, daß das rekombinante Plasmid in *Escherichia coli* NRRL-B-15989 enthalten ist.

26. Verfahren nach Anspruch 21, dadurch gekennzeichnet, daß das rekombinante Plasmid in *Escherichia coli* NRRL-B-15990 enthalten ist.

27. Verfahren nach Anspruch 21, dadurch gekennzeichnet, daß das rekombinante Plasmid in *E. coli* NRRL-B-15991 enthalten ist.

28. Verfahren nach Anspruch 21, dadurch gekennzeichnet, daß das lineare Fragment aus ERA103 ATCC 39824 B1 stammt, der mit der Endonuklease *Eco*RI, *Sph*I oder *Eco*RI und dann *Sph*I gespalten worden ist, und das Vektorplasmid aus der pOP203($A_2^+$), pBR322 und pKK223—3 umfassenden Gruppe ausgewählt ist, wobei das Vektorplasmid mit *Eco*RI gespalten und dann ligiert worden ist, um ein rekombinantes Plasmid bereitzustellen.

29. Verfahren nach Anspruch 21, dadurch gekennzeichnet, daß der Phage *Erwinia amylovora* lysiert und die Depolymerase eine Polysaccharidkapsel von *Erwinia amylovora* abbaut.

30. Verfahren nach Anspruch 22, dadurch gekennzeichnet, daß der *Erwinia amylovora* ATCC 39824 ist.

# EP 0 219 009 B1

**Revendications pour les Etats contractants: BE CH DE FR GB IT LI LU NL SE**

1. Plasmide recombinant qui peut exprimer une polysaccharide dépolymérase dans une bactérie contenant le plasmide, qui comprend:

(a) un segment linéaire d'un bactériophage qui infecte *Erwinia amylovora* pour produire la dépolymérase et qui code pour la dépolymérase; et

(b) un segment de plasmide vecteur transformable lié au segment linéaire pour donner le plasmide recombinant.

2. Le plasmide de la Revendication 1, caractérisé en ce que le segment linéaire provient du bactériophage ERA103 déposé en tant que ATCC 39824 B1.

3. Le plasmide recombinant de la Revendication 1 tel qu'il est porté dans *E. coli* NRRL-B-15989.

4. Le plasmide recombinant de la Revendication 1 tel qu'il est porté dans *E. coli* NRRL-B-15990.

5. Le plasmide recombinant de la Revendication 1 tel qu'il est porté dans *E. coli* NRRL-B-15991.

6. Le plasmide recombinant de la Revendication 1, caractérisé en ce que le segment linéaire provient de ERA103 déposé en tant que ATCC 39824 B1, clivé par une endonucléase *Eco*RI, *Sph*I, ou *Eco*RI et puis *Sph*I et le plasmide vecteur est choisi entre pOP203($A_2^+$), pBR322 et pKK223—3, clivé par *Eco*RI, qui a été lié au segment linéaire pour donner le plasmide recombinant.

7. Le plasmide recombinant de la Revendication 1, caractérisé en ce que la bactérie est *E. coli*.

8. Le plasmide de la Revendication 1, caractérisé en ce que le plasmide vecteur et le segment linéaire ont été clivés par *Eco*RI et liés.

9. Le plasmide de la Revendication 1, caractérisé en ce que le phage lyse *Erwinia amylovora* et en ce que la polysaccharide dépolymérase dégrade une capsule polysaccharidique autour d'*Erwinia amylovora*.

10. Le plasmide de la Revendication 9, caractérisé en ce que *Erwinia amylovora* est déposé en tant que ATCC 39824.

11. Une bactérie qui exprime une polysaccharide dépolymérase à cause d'un plasmide recombinant résident, caractérisée en ce que le plasmide recombinant comprend:

(a) un segment linéaire d'un bactériophage qui infecte *Erwinia amylovora* pour produire la dépolymérase et qui code pour la dépolymérase; et

(b) un segment de plasmide vecteur transformable lié au segment linéaire pour donner le plasmide recombinant.

12. La bactérie de la Revendication 11, caractérisée en ce que le segment linéaire provient du bactériophage ERA103 tel qu'il est porté dans ATCC 39824 B1.

13. La bactérie de la Revendication 11 contenant le plasmide recombinant tel qu'il est porté dans *Escherichia coli* NRRL-B-15990.

14. La bactérie de la Revendication 11 contenant le plasmide recombinant porté dans *Escherichia coli* NRRL-B-15991.

15. La bactérie de la Revendication 11 contenant le plasmide recombinant tel qu'il est porté dans *E. coli* NRRL-B-15989 (pour pSRQ52).

16. La bactérie de la Revendication 11, caractérisée en ce que le segment linéaire provient de ERA103 clivé par une endonucléase *Eco*RI, *Sph*I ou *Eco*RI et ensuite *Sph*I et le plasmide vecteur est choisi entre pOP203($A_2^+$), pBR322 et pKK223—3 clivé par *Eco*RI et qui a été lié au segment linéaire pour donner le plasmide recombinant.

17. La bactérie de la Revendication 11, qui est une souche de *Escherichia coli*.

18. La bactérie de la Revendication 11, caractérisée en ce que le plasmide vecteur et le segment linéaire ont été clivés par *Eco*RI et liés.

19. La bactérie de la Revendication 11, caractérisée en ce que le phage lyse *Erwinia amylovora* et en ce que la polysaccharide dépolymérase dégrade une capsule polysaccharidique autour d'*Erwinia amylovora*.

20. La bactérie de la Revendication 19, caractérisée en ce que *Erwinia amylovora* est ATCC 39824.

21. Une méthode pour produire une polysaccharide dépolymérise qui comprend:

(a) la fourniture d'une bactérie pour l'expression de la polysaccharide dépolymérase contenant un plasmide recombinant résident avec un segment linéaire provenant d'un bactériophage qui infecte *Erwinia amylovora* pour produire la dépolymérase et qui code pour la dépolymérase, et avec un segment de plasmide vecteur transformable qui a été lié au segment linéaire pour donner le plasmide recombinant;

(b) la culture de la bactérie dans un milieu de croissance pour produire la dépolymérase; et

(c) l'extraction de la dépolymérase de la bactérie et du milieu de croissance.

22. La méthode de la Revendication 21, caractérisée en ce que les cellules des bactéries sont dégradées et la dépolymérase est extraite des cellules dégradées.

23. La méthode de la Revendication 21, caractérisée en ce que le segment linéaire provient du bactériophage ERA103 déposé en tant que ATCC 39824 B1.

24. La méthode de la Revendication 21, caractérisée en ce que la bactérie est *Escherichia coli*.

25. La méthode de la Revendication 21, caractérisée en ce que le plasmide recombinant est porté dans *Escherichia coli* NRRL-B-15989.

26. La méthode de la Revendication 21, caractérisée en ce que le plasmide recombinant est porté dans *Escherichia coli* NRRL-B-15990.

27. La méthode de la Revendication 21, caractérisée en ce que le plasmide recombinant est porté dans *E. coli* NRRL-B-15991.

28. La méthode de la Revendication 21, caractérisée en ce que le segment linéaire provient de ERA103 ATCC 39824 B1 clivé par une endonucléase *Eco*RI, *Sph*I ou *Eco*RI et puis *Sph*I et le plasmide vecteur est choisi entre pOP203(A$_2$$^+$), pBR322 et pKK223—3 clivé par *Eco*RI et ensuite lié pour donner le plasmide recombinant.

29. La méthode de la Revendication 21, caractérisée en ce que le phage lyse *Erwinia amylovora* et en ce que la dépolymérase dégrade une capsule polysaccharidique de *Erwinia amylovora*.

30. La méthode de la Revendication 29, caractérisée en ce que *Erwinia amylovora* est ATCC 39824.

**Revendications pour l'Etat contractant: AT**

1. Procédé pour la préparation d'un plasmide recombinant qui peut exprimer une polysaccharide dépolymérase dans des bactéries contenant le plasmide, qui comprend:

(a) un segment linéaire d'un bactériophage qui infecte *Erwinia amylovora* pour produire la dépolymérase et qui code pour la dépolymérase; et

(b) un segment de plasmide vecteur transformable lié au segment linéaire pour donner le plasmide recombinant.

2. Le procédé de la Revendication 1, caractérisé en ce que le segment linéaire provient du bactériophage ERA103 déposé en tant que ATCC 39824 B1.

3. Le procédé de la Revendication 1, aboutissant à un plasmide recombinant tel qu'il est porté dans *E. coli* NRRL-B-15989.

4. Le procédé de la Revendication 1, aboutissant à un plasmide recombinant tel qu'il est porté dans *E. coli* NRRL-B-15990.

5. Le procédé de la Revendication 1, aboutissant à un plasmide recombinant tel qu'il est porté dans *E. coli* NRRL-B-15991.

6. Le procédé de la revendication 1, caractérisé en ce que le segment linéaire provient de ERA103 déposé en tant que ATCC 39824 B1, clivé par une endonucléase *Eco*RI, *Sph*I, ou *Eco*RI et puis *Sph*I et le plasmide vecteur est choisi entre pOP203(A$_2$$^+$), pBR322 et pKK223—3, clivé par *Eco*RI, qui a été lié au segment linéaire pour donner le plasmide recombinant.

7. Le procédé de la Revendication 1, caractérisé en ce que la bactérie est *E. coli*.

8. Le procédé de la Revendication 1, caractérisé en ce que le plasmide vecteur et le segment linéaire sont clivés par *Eco*RI et liés.

9. Le procédé de la Revendication 1, caractérisé en ce que le phage lyse *Erwinia amylovora* et en ce que la polysaccharide dépolymérase dégrade une capsule polysaccharidique autour d'*Erwinia amylovora*.

10. Le procédé de la Revendication 9, caractérisé en ce que *Erwinia amylovora* est déposé en tant que ATCC 39824.

11. Procédé pour la préparation d'une bactérie qui exprime une polysaccharide dépolymérase, caractérisé en ce qu'un plasmide recombinant est introduit dans ladite bactérie, où le plasmide recombinant comprend:

(a) un segment linéaire d'un bactériophage qui infecte *Erwinia amylovora* pour produire la dépolymérase et qui code pour la dépolymérase; et

(b) un segment de plasmide vecteur transformable lié au segment linéaire pour donner le plasmide recombinant.

12. Le procédé de la Revendication 11, caractérisé en ce que le segment linéaire provient du bactériophage ERA103 tel qu'il est porté dans ATCC 39824 B1.

13. Le procédé de la Revendication 11, caractérisé en ce que le plasmide recombinant est le plasmide tel qu'il est porté dans *Escherichia coli* NRRL-B-15990.

14. Le procédé de la Revendication 11, caractérisé en ce que le plasmide recombinant est le plasmide tel qu'il est porté dans *Escherichia coli* NRRL-B-15991.

15. Le procédé de la Revendication 11, caractérisé en ce que le plasmide recombinant est le plasmide tel qu'il est porté dans *Escherichia coli* NRRL-B-15989.

16. Le procédé de la Revendication 11, caractérisé en ce que le segment linéaire provient de ERA103 clivé par une endonucléase *Eco*RI, *Sph*I ou *Eco*RI et ensuite *Sph*I et le plasmide vecteur est choisi entre pOP203(A$_2$$^+$), pBR322 et pKK223—3 clivé par *Eco*RI et qui a été lié au segment linéaire pour donner le plasmide recombinant.

17. Le procédé de la Revendication 11, caractérisé en ce que la bactérie est une souche de *Escherichia coli*.

18. Le procédé de la Revendication 11, caractérisé en ce que le plasmide vecteur et le segment linéaire ont été clivés par *Eco*RI et liés.

19. Le procédé de la Revendication 11, caractérisé en ce que le phage lyse *Erwinia amylovora* et en ce que la polysaccharide dépolymérase dégrade une capsule polysaccharidique autour d'*Erwinia amylovora*.

20. Le procédé de la Revendication 19, caractérisé en ce que *Erwinia amylovora* est ATCC 39824.

21. Méthode pour la production d'une polysaccharide dépolymérase, qui comprend:

(a) la fourniture d'une bactérie pour l'expression de la polysaccharide dépolymérase contenant un

13

plasmide recombinant résident avec un segment linéaire provenant d'un bactériophage qui infecte *Erwinia amylovora* pour produire la dépolymérase et avec un segment de plasmide vecteur transformable qui a été lié au segment linéaire pour donner le plasmide recombinant;

(b) la culture de la bactérie dans un milieu de croissance pour produire la dépolymérase; et

(c) l'extraction de la dépolymérase de la bactérie et du milieu de croissance.

22. La méthode de la Revendication 21, caractérisée en ce que les cellules des bactériés sont dégradées et la dépolymérase est extraite des cellules dégradées.

23. La méthode de la Revendication 21, caractérisée en ce que le segment linéaire provient du bactériophage ERA103 déposé en tant que ATCC 39284 B1.

24. La méthode de la Revendication 21, caractérisée en ce que la bactérie est *Escherichia coli*.

25. La méthode de la Revendication 21, caractérisée en ce que le plasmide recombinant est porté dans *Escherichia coli* NRRL-B-15989.

26. La méthode de la Revendication 21, caractérisée en ce que le plasmide recombinant est porté dans *Escherichia coli* NRRL-B-15990.

27. La méthode de la Revendication 21, caractérisée en ce que le plasmide recombinant est porté dans *E. coli* NRRL-B-15991.

28. La méthode de la Revendication 21, caractérisée en ce que le segment linéaire provient de ERA103 ATCC 39824 B1 clivé par une endonucléase *Eco*RI, *Sph*I ou *Eco*RI et ensuite *Sph*I et le plasmide vecteur est choisi entre pOP203($A_2^+$), pBR322 et pKK223-3 clivé par *Eco*RI et ensuite lié pour donner le plasmide recombinant.

29. La méthode de la Revendication 21, caractérisée en ce que le phage lyse *Erwinia amylovora* et en ce que la dépolymérase dégrade une capsule polysaccharidique de *Erwinia amylovora*.

30. La méthode de la Revendication 22, caractérisée en ce que *Erwinia amylovora* est ATCC 39824.

FIG. 1

FIG. 2